# EUROPEAN PATENT SPECIFICATION

(11) **EP 2 515 811 B1**
(45) Date of publication and mention of the grant of the patent: **20.05.2015**
(21) Application number: 10798638.2
(22) Date of filing: 07.12.2010
(51) Int. Cl.: A61F 11/08

(54) **EAR PROTECTOR WITH AN ACOUSTIC DAMPING FILTER FOR HEARING PROTECTION AS WELL AS FILTER HOUSING FOR SUCH AN EAR PROTECTOR**
OHRENSCHUTZ MIT SCHALLDÄMPFUNGSFILTER FÜR GEHÖRSCHUTZ SOWIE FILTERGEHÄUSE FÜR EINEN DERARTIGEN OHRENSCHUTZ
PROTÈGE-OREILLE ÉQUIPÉ D'UN FILTRE D'AMORTISSEMENT ACOUSTIQUE POUR PROTÉGER L'AUDITION ET BOÎTIER DE FILTRE POUR UN PROTÈGE-OREILLE DE CE TYPE

(30) Priority: 22.12.2009 NL 2004003
(43) Date of publication of application: 31.10.2012
(73) Proprietor: Dynamic Ear Company B.V., 2629 HG Delft (NL)
(72) Inventor: WILMINK, Engbert, NL-2611PT Delft (NL)
(74) Representative: Jansen, Cornelis Marinus
(86) International application number: PCT/NL2010/050828
(87) International publication number: WO 2011/078660

(56) References cited:
- DE-B- 1 120 632
- US-A1- 2006 045 284
- US-A1- 2007 125 590

## Description

**Field of the invention:** The invention relates to ear protectors with an acoustic damping filter for hearing protection.

### Description of prior art:

The invention relates to an ear protector with an acoustic damping filter for hearing protection, comprising at least one acoustic element being exchangeable mounted in the filter and defining an acoustic channel extending from one side of the filter through the at least one acoustic element to the other side.

For protection of the ear against loud noises, acoustic damping filters are known providing a certain damping, many of them also dependent on frequency. Health and safety rules prescribe damping criteria for these acoustic damping filters, but also the damping should meet the requirements of the wearer.

Such an ear protector with an acoustic damping filter is disclosed in US 5.113.967. In this known ear protector the acoustic element can be substituted for one with a different damping parameter by pressing the acoustic element in the acoustic channel. Since a change of damping can only be realized by substituting filters, this known ear protector is missing the flexibility to create a filter that can fulfil most of different requirements.

US 2007125590 (A1) discloses a hearing protection device comprising a shell to be worn at least partially within a user's ear canal. The shell is provided with a receptacle having an outer opening towards ambience and an inner opening towards said ear canal. An acoustic filter element for attenuating sound comprising open-pore porous material is engaged within the receptacle in a detachable manner.

It is an object of the invention to provide an ear protector with an acoustic damping filter with the flexibility to fulfil most of different damping requirements and yet cheap to manufacture.

### Disclosure of the invention

According to the invention the filter is adapted to mount a number of interchangeable acoustic elements, the acoustic damping characteristics of the filter can be adapted by mounting acoustic elements with different damping parameters.

With this filter a cost effective basis is created for mounting acoustic damping elements with all kind of sound damping parameters, but also blocking impulse sound waves, enabling air transmission or closing, enabling water sealing or moister transfer, enabling visual identification, suitable for workers in food industry, etc.

The whole of the filter and the acoustic elements result into a certain acoustic sound transmission through the acoustic channel.

The filter comprises a first and a second acoustic channel extending from one side of the filter through the at least one acoustic element to the other side. A dimension of a passage of the second acoustic channel can be adjusted by positioning of a housing of the filter with respect to the at least one acoustic element.

In an embodiment, said second acoustic channel is designed for compensation of the loudness effect by less acoustic damping of low frequencies. Because of this effect, a specific (physically flat) attenuation will be experienced by the user to be higher for the lower frequency than for the higher frequencies. Although the user has a flat filter in his ears, he has the feeling that the lower frequencies are attenuated more. In order to compensate for this effect, said second acoustic channel is made to decrease the low frequency attenuation.

By choosing another form of said secondary acoustic channel, it can be used to equalize pressure differences between the inner and middle ear. This is for example useful for musicians playing wind instruments.

In an embodiment, a filter housing for such an ear protector is provided, wherein a wall of the housing is provided with identifier means for identifying the filter type. This identification can be realised without affecting the damping performance of the acoustic damping filter.

Further advantageous embodiments of the invention are described in other dependent claims.

### Brief description of the drawings

The invention will be further elucidated on the basis of embodiments represented in the drawing, in which:
Fig. 1 shows a sectional view of an ear protector with an acoustic damping filter for hearing protection according to a first embodiment of the invention;
Fig. 2 shows on enlarged scale a sectional view of the damping filter of the ear protector of fig. 1;
Fig. 3 shows a sectional view along lines III-III through a wall of the filter housing of the damping filter in fig.2;

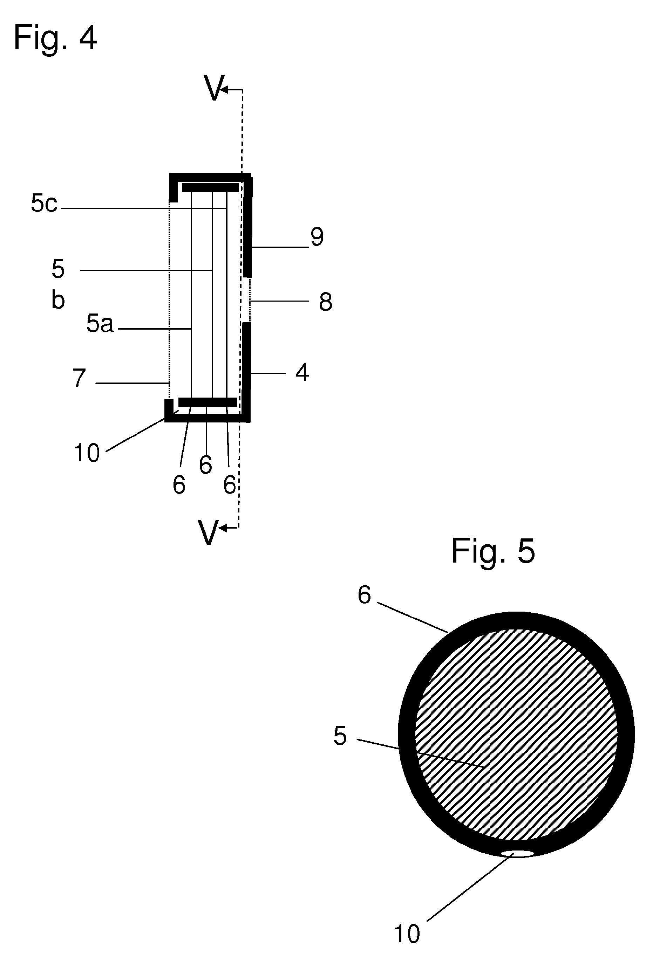

Fig. 4 shows on enlarged scale a sectional view of the damping filter of the ear protector of fig. 1 with a first and a second acoustic channel according to a second embodiment of the invention; and
Fig. 5 shows a sectional view along lines V-V through a wall of the filter housing of the damping filter in fig.4.

### Description of the embodiments

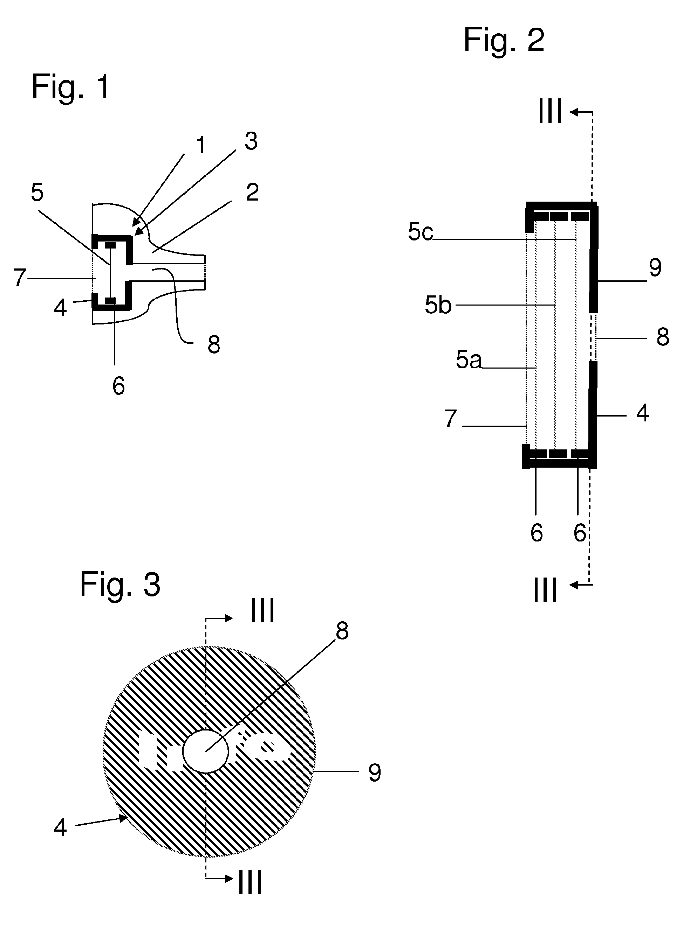

An ear protector 1, shown in fig.1, comprises a custom earpiece 2 with an acoustic damping filter 3 for hearing protection. The filter 3 may be part of the earpiece 2. In a preferred embodiment the filter is located in a filter housing 4 and comprises at least one acoustic element 5 being exchangeable mounted in a support 6 in the filter and that defines an acoustic channel extending from one side 7 of the filter through the at least one acoustic element 5 to the other side 8. The opening of the housing 4 at the side 7 is significant larger than at the side 8. The filter housing is adapted to mount a number of acoustic elements, fig.2 shows three acoustic elements 5a, 5b, 5c with different damping parameters, mounted for example by clamping in the supports 6. Mounting of other numbers of acoustic elements is possible. For easy mounting the filter housing is removable connected to the ear protector. For a quick selection of the elements, the damping parameter of an acoustic element is identified by the colour of the element. An acoustic element may comprise the combination of acoustic damping material and sealing material.

At least one of the acoustic elements may comprise an opening for less acoustic damping of low frequencies. Preferably the filter comprises at least two acoustic elements with an opening. The dimension of the passage of the acoustic channel can be adjusted by rotation of the elements with respect to each other. The diameter of the openings in the acoustic elements may differ.

The possibility of substitution and positioning of acoustic elements 5 in the filter 3, gives the ear protector the flexibility to fulfil most of different damping requirements.

As shown in fig. 3 in a wall 9 of the housing 4 at the location of the side 8, identifier means are provided for identifying the filter type and its characteristics. This identifier includes advantageous info relating the filter or the logo of the manufacturer.

As shown in fig.'s 4 and 5 in a second embodiment of the invention the filter 3 comprises a first and a second acoustic channel extending from one side of the filter to the other side. The first channel extends from the side 7 of the filter through the acoustic elements 5a, 5b, 5c to the other side 8. The second channel may pass an opening 10 in the housing 4, or alternatively (not shown) an opening in an edge of the elements 5. Said second acoustic channel being designed for less acoustic damping of low frequencies. For adjusting the damping characteristics the dimension of the acoustic channel can be varied by rotation of the housing 4 of the filter 3 with respect to the at least one acoustic element 5. Alternatively, if the second channel passes openings in an edge of the elements 5, the dimension of the passage of the second acoustic channel can be adjusted by rotation of the elements with respect to each other.

The detailed drawings, specific examples and particular formulations given, serve the purpose of illustration only. Furthermore, other substitutions, modifications, changes, and omissions may be made in the design, operating conditions, and arrangement of the exemplary embodiments without departing from the scope of the invention as expressed in the appended claims

## Claims

1. Ear protector (1) with an acoustic damping filter (3) for hearing protection, comprising at least one acoustic element (5) being exchangeable mounted in the filter (3) and defining an acoustic channel extending from one side of the filter through the at least one acoustic element (5) to the other side, wherein the filter (3) is adapted to mount a number of interchangeable acoustic elements (5), the acoustic damping characteristics of the filter (3) can be adapted by mounting acoustic elements (5) with different damping parameters **characterized in that** the filter (3) comprises a first and a second acoustic channel extending from one side of the filter (3) through the at least one acoustic element (5) to the other side wherein a dimension of a passage of the second acoustic channel can be adjusted by positioning of a housing (4) of the filter (3) with respect to the at least one acoustic element (5).

2. Ear protector (1) in accordance with claim 1, **characterized in that** the filter (3) is removable connected to the ear protector (1).

3. Ear protector (1) in accordance with claim 1 or 2, **characterized in that** said second acoustic channel is designed for compensation of the loudness effect by less acoustic damping of low frequencies.

4. Ear protector in accordance with claim 1 or 2, **characterized in that** said second acoustic channel is designed for equalizing pressure differences between the inner and middle ear.

5. Ear protector (1) in accordance with claim 3 or 4, **characterized in that** the dimension of the passage of the second acoustic channel can be adjusted by rotation of a housing (4) of the filter with respect to the at least one acoustic element (5).

6. Ear protector (1) in accordance with anyone of claims 3 to 5, **characterized in that** an edge of the at least acoustic element comprises an opening (10), being part of the passage of the second acoustic channel.

7. Ear protector in accordance with anyone of claims 3 to 6, **characterized in that** the housing of the filter comprises an opening, being part of the passage of the second acoustic channel.

8. Ear protector (1) in accordance with claim 3 or 4, **characterized in that** the filter comprises at least two acoustic elements, the dimension of the passage of the second acoustic channel can be adjusted by rotation of the elements with respect to each other.

9. Ear protector (1) in accordance with claim 3 or 4, **characterized in that** the filter comprises at least two acoustic elements with an opening, the dimension of the passage of the acoustic channel can be adjusted by rotation of the elements with respect to each other.

10. Ear protector (1) in accordance with any preceding claim, **characterized in that** the at least one acoustic element comprises acoustic damping material and sealing material.

11. Ear protector (1) in accordance with any preceding claim, **characterized in that** the damping parameter of an acoustic element is identified by the colour of the element.

12. Ear protector (1) in accordance with any preceding claim, further comprising a filter housing (4) for an acoustic damping filter (5) of the ear protector (1), , **characterized in that** a wall (9) of the housing (4) is provided with identifier means for identifying the filter type.

## Patentansprüche

1. Ohrenschutz (1) mit Schalldämpfungsfilter (3) für Gehörschutz, umfassend mindestens ein akustisches Element (5), das austauschbar in dem Filter (3) angebracht ist und einen akustischen Kanal definiert, verlaufend von einer Seite des Filters durch das mindestens eine akustische Element (5) zur anderen Seite, wobei der Filter (3) geeignet ist, um eine Anzahl austauschbarer akustischer Elemente (5) anzubringen, wobei die Schalldämpfungseigenschaften des Filters (3) durch Anbringen akustischer Elemente (5) mit unterschiedlichen Dämpfungsparametern angepasst werden können, **dadurch gekennzeichnet, dass** der Filter (3) einen ersten und einen zweiten akustischen Kanal umfasst, verlaufend von einer Seite des Filters (3) durch das mindestens eine akustische Element (5) zur anderen Seite, wobei eine Abmessung eines Durchgangs des zweiten akustischen Kanals durch Positionieren eines Gehäuses (4) des Filters (3) in Bezug auf das mindestens eine akustische Element (5) eingestellt werden kann.

2. Ohrenschutz (1) nach Anspruch 1, **dadurch gekennzeichnet, dass** der Filter (3) entfernbar mit dem Ohrenschutz (1) verbunden ist.

3. Ohrenschutz (1) nach Anspruch 1 oder 2, **dadurch gekennzeichnet, dass** der zweite akustische Kanal zum Kompensieren des Lautstärkeeffekts durch weniger Schalldämpfung von niedrigen Frequenzen konzipiert ist.

4. Ohrenschutz nach Anspruch 1 oder 2, **dadurch gekennzeichnet, dass** der zweite akustische Kanal zum Ausgleichen von Druckunterschieden zwischen dem Innen- und Mittelohr konzipiert ist.

5. Ohrenschutz (1) nach Anspruch 3 oder 4, **dadurch gekennzeichnet, dass** die Abmessung des Durchgangs des zweiten akustischen Kanals durch Drehen eines Gehäuses (4) des Filters in Bezug auf das mindestens eine akustische Element (5) eingestellt werden kann.

6. Ohrenschutz (1) nach einem der Ansprüche 3 bis 5, **dadurch gekennzeichnet, dass** ein Rand des mindestens einen akustischen Elements eine Öffnung (10) umfasst, die Teil des Durchgangs des zweiten akustischen Kanals ist.

7. Ohrenschutz nach einem der Ansprüche 3 bis 6, **dadurch gekennzeichnet, dass** das Gehäuse des Filters eine Öffnung umfasst, die Teil des Durchgangs des zweiten akustischen Kanals ist.

8. Ohrenschutz (1) nach Anspruch 3 oder 4, **dadurch gekennzeichnet, dass** der Filter mindestens zwei akustische Elemente umfasst, wobei die Abmessung des Durchgangs des Durchgangs des zweiten akustischen Kanals durch Drehen der Elemente zueinander eingestellt werden kann.

9. Ohrenschutz (1) nach Anspruch 3 oder 4, **dadurch gekennzeichnet, dass** der Filter mindestens zwei akustische Elemente mit einer Öffnung umfasst, wobei die Abmessung des Durchgangs des akustischen Kanals durch Drehen der Elemente zueinander eingestellt werden kann.

10. Ohrenschutz (1) nach einem der vorhergehenden Ansprüche, **dadurch gekennzeichnet, dass** das mindestens eine akustische Element Schalldämpfungsmaterial und Dichtungsmaterial umfasst.

11. Ohrenschutz (1) nach einem der vorhergehenden Ansprüche, **dadurch gekennzeichnet, dass** der Dämpfungsparameter eines akustischen Elements durch die Farbe des Elements identifiziert wird.

12. Ohrenschutz (1) nach einem der vorhergehenden Ansprüche, ferner umfassend ein Filtergehäuse (4) für einen Schalldämpfungsfilter (5) des Ohrenschutzes (1), **dadurch gekennzeichnet, dass** eine Wand (9) des Gehäuses (4) mit Identifikationsmitteln zum Identifizieren des Filtertyps versehen ist.

## Revendications

1. Protège-oreille (1) muni d'un filtre d'amortissement acoustique (3) destiné à protéger l'audition, qui comprend au moins un élément acoustique (5) échangeable monté dans le filtre (3) et définissant un canal acoustique qui s'étend d'un côté du filtre à l'autre côté, en passant par ledit élément acoustique (5), dans lequel le filtre (3) est adapté pour monter un certain nombre d'éléments acoustiques interchangeables (5), les caractéristiques d'amortissement acoustique du filtre (3) pouvant être adaptées en montant des éléments acoustiques (5) qui présentent des paramètres d'amortissement différents, **caractérisé en ce que** le filtre (3) comprend un premier et un second canaux acoustiques qui s'étendent d'un côté du filtre (3) à l'autre côté, en passant par ledit élément acoustique (5), et dans lequel une dimension d'un passage du second canal acoustique peut être ajustée en positionnant un logement (4) du filtre (3) par rapport audit élément acoustique (5).

2. Protège-oreille (1) selon la revendication 1, **caractérisé en ce que** le filtre (3) est relié de manière amovible au protège-oreille (1).

3. Protège-oreille (1) selon la revendication 1 ou 2, **caractérisé en ce que** ledit second canal acoustique est conçu pour compenser l'effet de volume sonore *(loudness)* par un amortissement acoustique moins fort des basses fréquences.

4. Protège-oreille selon la revendication 1 ou 2, **caractérisé en ce que** ledit second canal acoustique est conçu pour égaliser les différences de pression entre l'oreille interne et l'oreille moyenne.

5. Protège-oreille (1) selon la revendication 3 ou 4, **caractérisé en ce que** la dimension du passage du second canal acoustique peut être ajustée par la rotation d'un logement (4) du filtre par rapport audit élément acoustique (5).

6. Protège-oreille (1) selon l'une quelconque des revendications 3 à 5, **caractérisé en ce qu'**un bord dudit élément acoustique comprend une ouverture (10), qui fait partie du passage du second canal acoustique.

7. Protège-oreille selon l'une quelconque des revendications 3 à 6, **caractérisé en ce que** le logement du filtre comprend une ouverture, qui fait partie du passage du second canal acoustique.

8. Protège-oreille (1) selon la revendication 3 ou 4, **caractérisé en ce que** le filtre comprend au moins deux éléments acoustiques, la dimension du passage du second canal acoustique pouvant être ajustée par la rotation des éléments les uns par rapport aux autres.

9. Protège-oreille (1) selon la revendication 3 ou 4, **caractérisé en ce que** le filtre comprend au moins deux éléments acoustiques munis d'une ouverture, la dimension du passage du canal acoustique pouvant être ajustée par la rotation des éléments les uns par rapport aux autres.

10. Protège-oreille (1) selon l'une quelconque des revendications précédentes, **caractérisé en ce qu'**au moins un élément acoustique comprend un matériau d'amortissement acoustique et un matériau d'étanchéité.

11. Protège-oreille (1) selon l'une quelconque des revendications précédentes, **caractérisé en ce que** le paramètre d'amortissement d'un élément acoustique est identifié par la couleur de l'élément.

12. Protège-oreille (1) selon l'une quelconque des revendications précédentes, qui comprend en outre un logement de filtre (4) destiné à un filtre d'amortissement acoustique (5) du protège-oreille (1), **caractérisé en ce qu'**une paroi (9) du logement (4) est munie d'un moyen d'identification destiné à identifier le type de filtre.
